# EUROPÄISCHE PATENTANMELDUNG

(11) **EP 1 398 305 A1**
(43) Veröffentlichungstag der Anmeldung: **17.03.2004**
(21) Anmeldenummer: 03019808.9
(22) Anmeldetag: 30.08.2003
(51) Int. Cl.: C07C 17/12, C07C 17/395, C07C 17/23, C07C 1/26, C07C 25/125

(54) **Verfahren zur Hydrodechlorierung von kernchlorierten ortho-Xylolen**

(30) Priorität: 12.09.2002 DE 10242223
(71) Anmelder: Clariant GmbH, 65929 Frankfurt am Main (DE)
(72) Erfinder: Mack, Karl-Ernst, Dr., 65207 Wiesbaden (DE); Decker, Daniel, Dr., 65834 Liederbach a. Ts. (DE)

(57) **Zusammenfassung**

Verfahren zur Hydrodechlorierung von kernchlorierten o-Xylolen und Rückgewinnung von o-Xylol unter Bildung von Chlorwasserstoff wobei die kernchlorierten o-Xylole in der Gasphase an einem Edelmetall enthaltenden Katalysator bei einer Temperatur im Bereich von 220 bis 360°C hydriert werden. Der Katalysator enthält vorzugsweise Palladium oder Platin, insbesondere geträgertes Palladium oder Platin. Die kernchlorierten o-Xylole können einzeln oder als Gemische eingesetzt werden.

## Beschreibung

Kernchlorierte ortho-Xylole - insbesondere kernmonochlorierte o-Xylole - stellen wichtige Ausgangsstoffe zur Herstellung von Agro- und Pharmaprodukten dar.

Bei der Herstellung der kernmonochlorieren o-Xylole durch Chlorierung von o-Xylol entstehen mit zunehmendem Umsatz an o-Xylol unvermeidlich auch kerndichlorierte o-Xylole und in geringem Umfang auch kerntrichlorierte o-Xylole. Die kernpolychlorierten o-Xylole müssen wegen ihrer geringen Wertigkeit häufig als Abfälle beseitigt werden. Dies erfordert z.B. bei der Verbrennung einen besonders hohen Aufwand wegen der Gefahr der Dioxinbildung.

Darüber hinaus wird von den kernmonochlorierten o-Xylolen häufig nur eines der beiden entstehenden Isomeren technisch weiter verwendet, so dass jenes Isomere mit geringerem Wert ebenfalls als Abfall angesehen und beseitigt werden muss. Häufig ist das 4-Chlor-1,2-di-methylbenzol das begehrtere Isomere. Das 3-Chlor-1,2-dimethylbenzol wird daher z.B. durch Destillation vom gewünschten Isomeren abgetrennt, häufig wie die kernpolychlorierten o-Xylole durch Verbrennung beseitigt und erhöht somit den unerwünschten Mehrverbrauch des Einsatzmaterials o-Xylol.

Da im Normalfall der Chlorierung von o-Xylol in Gegenwart von Lewis-Säuren die beiden Isomeren 4-Chlor- und 3-Chlor-1,2-dimethylbenzol in einem Verhältnis von kleiner 1,5 : 1 (entsprechend kleiner 60 % : 40 %) entstehen wurden erhebliche Anstrengungen unternommen durch Zusatz von Co-Katalysatoren eine Verschiebung des Isomerenverhältnisses in die Richtung des jeweils gewünschten Isomeren zu erhalten.

Als wirksame Co-Katalysatoren zur Verbesserung der Bildung von 4-Chlor-1,2-dimethylbenzol wurden z.B. die jedoch als hoch toxisch geltende Verbindungsklasse der Thianthrene (US-A-4,190, 609) oder aber die wegen sehr anspruchsvoller Substituentenmuster schwer zugängliche Verbindungen aus der Klasse der Thiazepine oder Thiazocine(WO 02/14245) genannt. Auch in den günstigsten Fällen der Verschiebung der Isomerenverhältnisse fallen aber dennoch die unerwünschten Isomeren ein einer Menge von mindestens 25 Gew.-% bezogen auf die Gewichtsmenge des gewünschten Isomeren an.

Weil weiterhin bei der Chlorierung von o-Xylol mit zunehmendem Umsatz zwangsläufig auch die unerwünschten kernpolychlorierten Produkte anfallen, wird der Umsatz an o-Xylol häufig begrenzt. Dies hat zur Folge, dass nicht umgesetztes o-Xylol unter relativ hohen Destillationsaufwand aus dem Reaktionsgemisch zurückgewonnen werden muss.

Es bestand daher die Aufgabe, aus ökonomischen und ökologischen Gründen ein Verfahren bereitzustellen, das es erlaubt, 4-Chlor-1,2-dimethylbenzol und/oder 3-Chlor-1,2-dimethylbenzol weitgehend ohne Zwangsanfall des jeweils unerwünschten Isomeren und der kernpolychlorierten o-Xylolen herzustellen.

Diese Aufgabe wurde auf überraschend einfache Weise dadurch gelöst, dass die jeweiligen Zwangsanfallprodukte isoliert und durch Behandeln mit Wasserstoff an einem Edelmetall enthaltenden Katalysator bei erhöhter Temperatur zu o-Xylol und Chlorwasserstoff umgewandelt werden wodurch o-Xylol wieder der Chlorierungsreaktion zugeführt und der entstehende Chlorwasserstoff zu technisch wertvoller Salzsäure aufgearbeitet werden kann.

Die Erfindung betrifft somit ein Verfahren zur Hydrodechlorierung von kernchlorierten o-Xylolen in der Gasphase an einem Edelmetall enthaltenden Katalysator unter Rückgewinnung von o-Xylol und unter Bildung von Chlorwasserstoff bei einer Temperatur im Bereich von 220 bis 360°C. Die als Nebenprodukt angefallenen kernchlorierten o-Xylole können dabei einzeln oder als Gemische eingesetzt werden.

Die Hydrodechlorierung von polychlorierten Benzolderivaten ist bekannt. Umsetzungen in der Flüssigphase z.B. mit Raney-Nickel und Wasserstoff (Chem. Ber. 91 (1958), 1376) oder z.B. mit Palladium auf Kohle (US-5,177,268) erfolgen dabei allgemein bei Temperaturen um 100°C unter Überdruck von Wasserstoff, häufig unter Verwendung eines Lösemittels und nachteilhafterweise unter Zusatz von Basen zum Neutralisieren des entstehenden Chlorwasserstoffs. Die dabei entstehenden Chloride müssen abgetrennt und beseitigt, das Lösemittel gegebenenfalls zurückgewonnen werden. Dies trifft auch zu für das Beispiel der Hydrodechlorierung von 4-Chlor-1,2-di-methylbenzol mit Palladium auf Kohle in wässriger Natronlauge bei 110°C und 4 bar Wasserstoffdruck zu, bei dem jedoch erhebliche Mengen an unerwünschten Nebenprodukten entstehen. Die Rückgewinnung von o-Xylol gelingt hierbei nur zu weniger als 40 % (Tetrahedron 55;51;1999; 14763).

Der Zusatz von Base kann häufig umgangen werden, indem in der Gasphase gearbeitet wird. Hier ergibt sich jedoch das Problem, dass aus thermodynamischen Gründen im Temperaturbereich unter 300°C bevorzugt Kernhydrierung stattfindet unter Bildung der entsprechenden Cyclohexanderivate (Ullmann's Encyclopedia of Industrial Chemistry, 5^{th} Edition, Volume A8, p. 211 (1987)). Typischerweise wird an einem palladiumgeträgerten Katalysator bei Temperaturen bis 200°C z.B. Trichlorbenzol vollständig dechloriert und zu Cyclohexan hydriert (US-5,625,110).

Die nachteilhafte Hydrierung des Benzolkerns kann bekanntermaßen durch Anwendung hoher Temperaturen vermindert werden wie z.B. in US-A-5,552,549 bei der Hydrodechlorierung von Polychlorbenzolen bei Temperaturen um 400°C. Hierbei wird aber allerdings keine vollständige Dechlorierung zu Benzol erreicht.

Überraschenderweise wurde nun gefunden, dass die Hydrodechlorierung von kernchlorierten o-Xylolen in der Gasphase im Temperaturbereich von 220 bis 360°C an Edelmetallkatalysatoren in hoher Ausbeute um 90 % und mit langen Standzeiten der Katalysatoren durchgeführt werden kann. Dies war deshalb besonders überraschend, da gerade in jenem Temperaturbereich um 300°C erfolgreich gearbeitet wird in welchem aufgrund der relativ niedrigen Temperatur einerseits noch ein erhebliches Ausmaß der Bildung von 1,2-Dimethylcyclohexan zu erwarten gewesen wäre, andererseits eine vollständige Dechlorierung besonders von kernpolychlorierten o-Xylolen noch nicht zu erwarten war.

Der Katalysator enthält als Edelmetalle vorzugsweise Palladium und/oder Platin oder Verbindungen dieser Metalle bevorzugt enthält er Palladium, geträgert auf einem oxidischen Material wie Aluminiumoxid oder Siliciumoxid oder Kohle. Besonders bevorzugt ist die Verwendung von Palladium enthaltenden Katalysatoren auf Kohle.

Der Gehalt an Edelmetall auf dem Trägermaterial kann in weiten Bereichen von 0,01 bis 5 Gew.-% variiert werden. Bevorzugt ist ein Gehalt von 0,1 bis 3 Gew.-%, besonders bevorzugt 0,5 bis 1,5 Gew.-%.

Die stündliche Beladung des Katalysators mit Ausgangsmaterial kann im Bereich von 0,05 bis 4 Litern Flüssigkeit pro Liter Katalysator betragen. Zweckmäßig ist eine stündliche Beladung von 0,1 bis 1 Liter an Flüssigkeit, besonders günstig eine solche von 0,2 bis 0,5 Litern pro Liter Katalysator.

Das molare Verhältnis von mono- zu polykernchlorierten o-Xylolen kann im Ausgangsmaterial jeden beliebigen Wert annehmen. Bevorzugt ist das beim Chlorierungsverfahren von o-Xylol unter ökonomischen Gesichtspunkten anfallende Gemisch mit Verhältnissen von 5 : 1 bis 1 : 2.

Die Menge an zugeführtem Wasserstoff soll mindestens äquimolar dem Molgehalt an Chlor im Ausgangsmaterial entsprechen. Zweckmäßig ist die Verwendung eines Überschusses an Wasserstoff, der die 3 bis 30-fache Molmenge, insbesondere die 5- bis 20-fache Molmenge betragen kann und in Abhängigkeit von der Aktivität des Katalysators und gegebenenfalls dem Grad der Verdünnung des Einsatzgemischs aus kernchlorierten o-Xylolen und Wasserstoff mit einem Inertgas wie z.B. Stickstoff im Einzelfall zu ermitteln ist.

Die Hydrodechlorierung kann unter normalem oder erhöhtem Druck durchgeführt werden. Aus technische und apparatebaulichen Gründen ist aber die Fahrweise unter Normaldruck zu bevorzugen.

Die im Katalysatorbett herrschende Temperatur kann im Bereich von 220°C bis 360°C variiert werden. Sie soll aber mindestens so hoch sein, dass keine Kondensation der kernchlorierten o-Xylole im Reaktor erfolgt. Bevorzugt ist der Temperaturbereich von 260 bis 350°C, besonders bevorzugt 280 bis 330°C.

Das erfindungsgemäße Verfahren wird z.B. in der Weise durchgeführt, dass o-Xylol in einer der eingangs beschriebenen Weisen chloriert wird. Das Reaktionsgemisch wird dann destillativ aufgetrennt in das gewünschte Produkt und das unerwünschte kernmonochlorierte o-Xylol. Aus dem Sumpf dieser Destillation werden die kerndichlorierten o-Xylole isoliert und entweder getrennt oder im Gemisch mit den als unerwünschten Nebenprodukt angefallenen kernmonochlorierten o-Xylol als dampfförmiges Einsatzmaterial mit Wasserstoff in oben beschriebener Weise über den Katalysator geleitet. Aus dem Reaktionsgemisch wird durch Kondensation o-Xylol in hoher Reinheit von > 95 %, insbesondere von 97 bis 99 %, und abhängig von der Güte der Kondensation in Ausbeuten von über 85 %, insbesondere 90 bis 95 %, gewonnen. Der im Abgas enthaltene Chlorwasserstoff wird in Wasser zu Salzsäure absorbiert. Die Standzeit des Katalysators beträgt unter Einhaltung der oben angegebenen bevorzugten Beladungsgrenzen mit Einsatzmaterial mindestens 700 Stunden, bevor er durch eine oder mehrere Regenerierungen mit geringen Luftmengen bei Reaktionstemperatur stets seine ursprüngliche Aktivität nahezu vollständig wiedererlangt.

Aufgrund der Wiederverwendung des zurückgewonnenen o-Xylols gelingt es, nahezu quantitative Ausbeuten des jeweils gewünschten kernmonochlorierten o-Xylols zu erzielen unter Erzeugung von geringsten Abfallmengen. Weiterhin erlaubt das erfindungsgemäße Verfahren auch je nach ökonomischen Gesichtspunkten o-Xylol bei der Chlorierung stets vollständig umzusetzen und somit vorteilhaft auf dessen destillative Rückgewinnung aus den Reaktionsgemisch verzichten zu können.

Die folgenden Beispiele sollen das erfindungsgemäße Verfahren erläutern, ohne es darauf einzuschränken.

### Beispiel 1

In ein senkrecht stehendes Glasrohr (Durchmesser 3,5 cm) in einem elektrisch beheizten Ofen werden 50 ml (ca. 25 g) eines Katalysators mit 1 Gew.-% Palladium auf gekörnter Kohle angebracht. Die Temperatur im Katalysatorbett wird unter Überleiten einer Gemischs aus je 40 l/h Wasserstoff und Stickstoff auf 290°C gebracht. Nach einer weiteren Stunde unter diesen Bedingungen werden zusätzlich kontinuierlich 15 g/h eines Gemischs aus nahezu gleichen Anteilen aus 4-Chlor-1,2-dimethylbenzol und 3-Chlor-1,2-dimethylbenzol dosiert. Die Verdampfung erfolgt dabei auf einer Schicht aus Glaskörpern oberhalb des Katalysators. Durch die sofort einsetzende Reaktion stellt sich eine Temperatur von 310°C im Katalysator ein. Der Reaktoraustrag wird auf ca. 25°C abgekühlt wobei eine farblose Flüssigkeit kondensiert. Die gaschromatographische Analyse ergibt o-Xylol in einer Reinheit von über 98 %. Es fallen über einen Zeitraum von 680 Stunden durchschnittlich 10 g Kondensat entsprechend einer Ausbeute um 90 % o-Xylol an.
Eine leicht abnehmende Katalysatoraktivität nach etwa 500 Stunden Betriebsdauer kann durch eine Erhöhung des Wasserstoffanteils im Gasstrom auf 50 l/h ausgeglichen werden.

Dieses Beispiel zeigt, dass sowohl das 4-Chlor- als auch das 3-Chlor-Isomere in gleicher Qualität dechloriert wird.

### Beispiel 2

Das Beispiel 1 wird wiederholt, wobei nun aber 15 g/h eines Gemischs aus 20 Gew.-% 4-Chlor-1,2-dimethylbenzol und 80 Gew.-% eines Isomerengemischs aus kerndichlorierten o-Xylolen dosiert wird. Die Temperatur stellt sich auf 320°C ein. Während 240 h bei gleichbleibender Katalysatoraktivität werden stündlich durchschnittlich 10 g o-Xylol in hoher Reinheit (> 98.5 %) entsprechend einer Ausbeute von ca. 92 % erhalten.

Dieses Beispiel zeigt, dass kernmono- und kerndichlorierte o-Xylole in gleicher Qualität dechloriert werden.

### Beispiel 3

Dieses Beispiel erläutert den Vorteil der erfindungsgemäßen Hydrodechlorierung in Zusammenhang mit der Chlorierung von o-Xylol.

In ein Doppelmantelgefäß mit 15 cm Innendurchmesser und 90 cm Höhe werden 6784 g (64 mol) o-Xylol gegeben, 3,4 g des in EP-A-0 173 222 beschriebenen Co-Katalysators tetrachloriertes 2,8-Dimethylphenoxathiin darin aufgelöst und bei 20°C am Boden des Reaktors innerhalb von 5 Stunden 4544 g (64 mol) Chlor über eine Glasfritte eingeleitet. Um die Glasfritte herum befindet sich eine Schüttung aus Eisenringen. Die Temperatur im Reaktor wird durch Kühlen auf 20°C gehalten.
Die gaschromatographische Analyse der 8900 g Reaktionsaustrag ergab 6,9 % o-Xylol, 65,6 % 4-Chlor-1,2-dimethylbenzol, 21,4 % 3-Chlor-1,2-dimethylbenzol, 5,5 % dichloriertes o-Xylol und 0,3 % Hochsieder. Daraus ergibt sich eine Ausbeute an 4-Chlor-1,2-dimethylbenzol, bezogen auf umgesetztes o-Xylol von 71,5 %.
Durch destillative Aufarbeitung werden als Destillate 1900 g 3-Chlor-1,2-dimethylbenzol sowie 490 g dichloriertes o-Xylol neben 30 g Hochsiedern als Sumpf erhalten.
Das Gemisch der Destillate (2390 g) wird analog zum Beispiel 1 während einer Dosierzeit von 160 Stunden hydrodechloriert. Es fallen 1560 g o-Xylol mit einer Reinheit von 98,5 % an und können je nach Bedarf nach einer destillative Reinigung wieder der Chlorierungsreaktion zugeführt werden.
Aufgrund der Rückgewinnung von o-Xylol verbessert sich die Ausbeute an 4-Chlor-1,2-dimethylbenzol von 71,5 auf über 95 %.

## Patentansprüche

1. Verfahren zur Hydrodechlorierung von kernchlorierten o-Xylolen und Rückgewinnung von o-Xylol unter Bildung von Chlorwasserstoff **dadurch gekennzeichnet, dass** die kernchlorierten o-Xylole in der Gasphase an einem Edelmetall enthaltenden Katalysator bei einer Temperatur im Bereich von 220 bis 360°C hydriert werden.

2. Verfahren gemäß Anspruch 1, **dadurch gekennzeichnet, dass** der Katalysator Palladium oder Platin, insbesondere geträgertes Palladium oder Platin enthält.

3. Verfahren nach mindestens einem der vorhergehenden Ansprüche, **dadurch gekennzeichnet, dass** als Träger für das Edelmetall oxidische Materialien wie Aluminiumoxid oder Siliciumoxid oder aber Kohle, bevorzugt Kohle verwendet wird.

4. Verfahren nach mindestens einem der vorhergehenden Ansprüche, **dadurch gekennzeichnet, dass** die Menge an zugeführtem Wasserstoff mindestens äquimolar dem Molgehalt im Ausgangsmaterial entspricht.

5. Verfahren nach mindestens einem der vorhergehenden Ansprüche, **dadurch gekennzeichnet** die kernchlorierten o-Xylole einzeln oder als Gemische eingesetzt werden.

6. Verfahren nach mindestens einem der vorhergehenden Ansprüche, **dadurch gekennzeichnet, dass** die Hydrierungsreaktion unter Normaldruck durchgeführt wird.
